# EUROPÄISCHE PATENTSCHRIFT

(11) **EP 0 342 617 B1**
(45) Veröffentlichungstag und Bekanntmachung des Hinweises auf die Patenterteilung: **28.07.1993**
(21) Anmeldenummer: 89108798.3
(22) Anmeldetag: 17.05.1989
(51) Int. Cl.: A61K 7/06, A61K 31/495

(54) **Verwendung von Trequinsin und kosmetische Mittel auf Basis dieser Verbindung**
Use of trequinsin and cosmetic preparations based on same
Utilisation de la trequinsine et préparations cosmétiques la contenant

(30) Priorität: 19.05.1988 DE 3816995
(43) Veröffentlichungstag der Anmeldung: 23.11.1989
(73) Patentinhaber: HOECHST AKTIENGESELLSCHAFT, 65926 Frankfurt am Main (DE)
(72) Erfinder: Rupp, Richard Helmut, Dr., D-6240 Königstein/Taunus (DE); Lal, Bansi, Dr., Bombay 400 080 (IN)

(56) Entgegenhaltungen:
- EP-A- 0 211 268
- DE-A- 3 601 739
- Journal of Medicinal Chemistry, Band 27, Nr. 11, November 1984, Seiten 1470-1480, American Chem. Soc., Washington, US; B. LAL et al.; "Trequinsin, a potent new natihypertensive vasodilator in the series of 2-(arylimino)-3-alkyl-9,10-dimethoxy-3,4,6,7-tetrahydro-2H-pyrimido(6,1-a)isoquinolin-4-ones"
- Archives of Dermatology, Band 120, April 1984, Seiten 457-463, Chicago, US; V.C. WEISS et al.: "Alopecia areata treated with topical minoxidil"

## Beschreibung

Die vorliegende Erfindung betrifft die Verwendung von Trequinsin und kosmetische Mittel auf Basis dieser Verbindung. (Trequinsin = 9,10-Dimethoxy-2-mesitylimino-3-methyl-2,3,6,7-tetrahydro-4H-pyrimido-(6,1-α)-isochinolin-4-on-hydrochlorid) Aus dem Verlust von Haaren ergibt sich Alopezie oder Kahlheit. Dermatologen unterscheiden verschiedene Arten von Haarverlust (z.B. Alopecia areata, Alopecia totalis oder androgenetische Alopezie), wobei der bei weitem am meisten verbreitete als androgenetische Alopezie oder "männliche Alopezie- oder Kahlheitsmuster" bekannt ist. Obwohl sich diese Art von Haarverlust weitgehend auf Männer beschränkt, ist sie bei Frauen nicht unbekannt. Das Alopezie- oder Kahlheitsleiden ist Folge einer Faktorenverknüpfung: (1) Umwandlung von Haaren vom Terminal- zum Flaumzustand, (2) größere Anzahl von telogenen Haaren - wovon einige abgestoßen wurden und (3) Verlust von Haarwurzeln. Über die Ursachen des männlichen Kahlheitsmusters ist sehr wenig bekannt, obwohl man vermutet, daß dieses genetischen oder hormonellen Ursprung haben könnte. Zum gegenwärtigen Zeitpunkt versucht man, das männliche Alopeziemuster entweder einerseits über nichtmedikamentöse Ansätze wie Haartransplantation, Massage unter Ultraviolettbestrahlung, psychiatrische Behandlungs- und Übungstherapie oder andererseits durch medikamentöse Behandlung zu kurieren. Über die Nichtmedikamentösen Lösungsansätze für das Problem sagt man, sie seien entweder allgemein unwirksam oder im Falle der Transplantation zu kostspielig, zeitaufwendig und unpraktisch. Für den Fall der medikamentösen Therapie hat man viele Arten therapeutischer Medikamente, die von Vitaminen zu Hormonen oder Diphenylhydantoin und Streptomycin reichen, ausprobiert, und erst in jüngster Zeit hat es dort ein Anzeichen eines bescheidenen Erfolgs gegeben. Unter den Behandlungen, die zu der bescheidenen Hoffnung Anlaß geben, daß durch ihre äußerliche Anwendung auf die Kopfhaut eines an männlichem Kahlheitsmuster Leidenden wieder Haare wachsen, gehört die Verwendung (1) einer Östradiol oder Oxandrolon enthaltene Mikroemulsionscreme, oder (2) organisches Silizium oder (3) Minoxidil.

Überraschenderweise wurde nun gefunden, daß Trequinsin ein außerordentlich wirksames Mittel zur Umwandlung von Flaumhaar in terminales Haar, zur Beschleunigung des terminalen Haarwuches und zum Anhalten des Haarverlustes, womit sie mögliche Mittel zur Behandlung, zum Anhalten und zur Umkehr von Alopezie (z.B. Alopecia totalis, Alopecia areata), insbesondere des männlichen Alopeziemusters (androgenetische Alopezie), darstellen. Pyrimido-(6,1-a)-isochinolin-4-one, zu denen auch das Trequinsin gehört sind bereits in der DE-OS 27 20 085 und der DE-OS 28 01 289 beschrieben, wo allerdings nur ihre Wirksamkeit zur Bekämpfung der Hypertonie, von Bronchospasmen und von Allergien Erwähnung findet.

Erfindungsgegenstand ist deshalb die Verwendung von 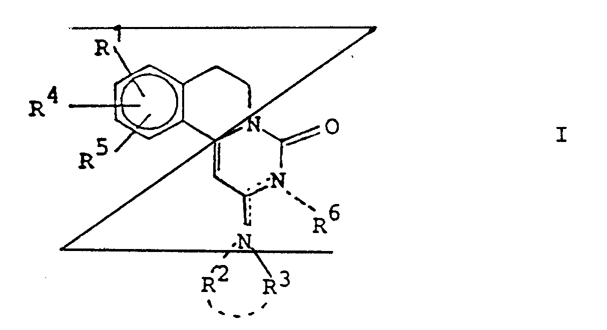
Trequinsin zur Herstellung einen Arzneimittels zur Verhinderung von Haarausfall.

An Stelle des Trequinsins kann auch eines seiner Salze mit anderen Salzbildnern verwendet werden.

Als Salze des Trequinsins mit anderen Salzbildnern gemäß vorliegender Erfindung seien beispielsweise solche von anorganischen oder organischen Säuren erwähnt, z.B., Hydrobromide, Sulfate, Phosphate, Acetate, Oxalate, Tartrate, Citrate, Maleate oder Fumarate.

Geeignete quaternäre Ammoniumsalze des Trequinsins sind z.B. die von Alkylhalogeniden abgeleiteten Salze wie Methyljodide.

Die Herstellung der erfindungsgemäß verwendbaren Verbindungen ist z.B. in der DE-OS 27 20 085 und DE-OS 28 01 289, auf die ausdrücklich Bezug genommen wird, beschrieben.

Die erfindungsgemäße Verwendung der Verbindungen der Formel I kann prinzipiell bei allen Säugetieren erfolgen; besondere Bedeutung besitzt sie für den Menschen. Eine Verbindung der Formel I wird vorzugsweise äußerlich auf die Säugetierhaut verabreicht.

Zur vorliegenden Erfindung gehören weiterhin kosmetische Mittel, insbesondere solche, die für die äußerliche und lokale Anwendung geeignet sind. Eine erfindungsgemäße Verbindung wird gemeinsam mit einem pharmazeutisch unbedenklichen, für äußerliche Anwendung geeigneten Träger zur Zubereitung herkömmlicher medizinischer Zubereitungen wie z.B. Lösungen, Suspensionen, Emulsionen, Pasten, Salben, Gelees, Cremes, Lotionen, Puder, Seifen, tensidhaltige Reinigungspräparate, Öle und Sprays verwendet. Zusätzlich zu dem Wirkstoff werden der Zubereitung beliebige übliche Trägerstoffe und Hilfsstoffe zugesetzt.

## Patentansprüche

1. Verwendung von Trequinsin zur Herstellung von Arzneimitteln zur Verhinderung von Haarausfall.

2. Verwendung von Trequinsin gemäß Anspruch 1 zur Herstellung von Arzneimitteln zur Verhinderung von durch androgenetische Alopezie, Alopecia totalis oder Alopecia areata bedingtem Haarausfall.

3. Kosmetisches Mittel zur Stärkung des Haarwuchses, dadurch gekennzeichnet, daß es Trequinsin enthält.

4. Kosmetisches Mittel gemäß Anspruch 3, dadurch gekennzeichnet, daß es in Form einer Lösung, einer Suspension, einer Emulsion, einer Paste, einer Salbe, eines Gelees, einer Creme, einer Lotion oder eines Puders vorliegt.

## Claims

1. The use of trequinsin for the preparation of medicaments for preventing hair loss.

2. The use of trequinsin as claimed in claim 1 for the preparation of medicaments for the prevention of hair loss caused by androgenetic alopecia, alopecia totalis or alopecia areata.

3. A cosmetic agent for strengthening hair growth, which contains trequinsin.

4. A cosmetic agent as claimed in claim 3, which is in the form of a solution, a suspension, an emulsion, a paste, an ointment, a gel, a cream, a lotion or a powder.

## Revendications

1. Utilisation de la tréquinsine pour la fabrication de médicaments pour empêcher la chute des cheveux.

2. Utilisation de la tréquinsine selon la revendication 1, pour la fabrication de médicaments pour empêcher la chute des cheveux due à l'alopécie androgénique, l'alopécie totale ou l'alopécie en aires.

3. Produit cosmétique pour stimuler la croissance capillaire, caractérisé en ce qu'il contient de la tréquinsine.

4. Produit cosmétique selon la revendication 3, caractérisé en ce qu'il se trouve sous forme d'une solution, d'une suspension, d'une émulsion, d'une pâte, d'une pommade, d'une gelée, d'une crème, d'une potion ou d'une poudre.
